# EUROPEAN PATENT APPLICATION

(11) **EP 4 389 009 A1**
(43) Date of publication of application: **26.06.2024**
(21) Application number: 22215145.8
(22) Date of filing: 20.12.2022
(51) Int. Cl.: A61B 6/03, A61B 6/00

(54) **DIGITAL TOMOSYNTHESIS IMAGING USING OPTICAL FIBER-BASED PATIENT 3D POSITIONING IN STANDARD RADIOGRAPHY SYSTEM**

(71) Applicant: Koninklijke Philips N.V., 5656 AG Eindhoven (NL)
(72) Inventor: CHAUDHURY, Sudipta, Eindhoven (NL); CHAKRABARTI, Biswaroop, Eindhoven (NL); SOUBHIK, Paul, 5656AG Eindhoven (NL); VON BERG, Jens, Eindhoven (NL); BRUECK, Heiner Matthias, Eindhoven (NL); GOOßEN, Andre, Eindhoven (NL)
(74) Representative: Philips Intellectual Property & Standards

(57) **Abstract**

A system (SYS) and related method for enabling tomographic imaging with a non-rotational radiography imaging apparatus (IA). An input interface (IN) receives, whilst there is relative motion between patient (PAT) to be imaged and a detector (DM) or X-ray source (XS) of the imaging apparatus, i) 3D position measurement readings acquired by an optical fiber-based system (OFS) via a set of optical-fiber based sensors (Si) arranged, for example, at the patient. The measurement readings are relatable to poses assumable by the patient during motion relative to the detector. Projection imagery (λ) acquirable by the imaging apparatus of the patient during, or before and after, such relative motion is also received. A projection direction determiner (PDD) determines projection direction data (α) for the projection imagery (λ) based on the 3D position measurement readings (P). The projection imagery associated with the projection direction data may be used for tomography.

## Description

### FIELD OF THE INVENTION

The invention relates to a system for facilitating tomographic imaging with a radiography imaging apparatus, an imaging arrangement including such a system, a wearable for use with such a system, to related method, to a computer program element and to a computer readable medium.

### BACKGROUND OF THE INVENTION

Medical imaging is among the most important tools in the medical field. Medical imaging reveals imagery from within the patient's body in order to inform medial tasks precisely, including diagnosis, therapy, planning, control operations of other medical equipment and many others.

X-ray based imaging is a mainstay of medical imaging. Radiography is one such example, a time-honored approach used to this day extensively since its inception more than a 100 years ago. Radiography is projection imaging, so has no depth of field. In some applications, this limitation is more than compensated for by the simple and cost-effective setup. What is more, in some applications, this projection-only imaging is more than enough for the purpose, such as in bone fracture detection, in trauma settings and others.

However, for many other applications, more detailed spatio-structural information is required and there is the need for image information along the third spatial dimension. This is because intervening structures, as they are encountered in purely projection-based imaging, may on occasion cause occlusion of crucial details.

Much different from radiography, tomography is a medical imaging technique where multi-directional X-ray projection imagery is acquired around the region of interest ("ROI") and is computationally combined to reveal 3D volumes of sectional slices of the ROI, such as of the lungs, bone, the heart, abdominal organ, etc, whatever the medical task at hand. Such tomographic volumes afford in some cases, such as in full angular CT, full depth of view and a maximum of spatial information from all directions. Limited angle tomography ("tomosynthesis") where projection imagery of less (sometimes way less) than 180° is acquired also add some depth of field information (sometimes called "2.5D" imaging as opposed to 3D imaging such as CT), but to a lesser extent, which, however, is again sufficient in some cases, such as in dentistry or mammography, etc.

Tomography/tomosyntheses can be used in diagnosing different abnormalities/conditions which include thorax imaging for pulmonary nodule detection, head and neck imaging for paranasal sinuses, dental imaging for improved spatial resolution, musculoskeletal imaging for high-resolution imaging of complex fractures and some emergency situations. For example, tomography/tomosyntheses provides much improved visualization of pathological lesion(nodule) in lungs using digital tomosynthesis over projection only radiography.

Useful and desirable as (X-ray) tomographic imaging (a shorthand herein for CT or tomosynthesis) is, it does add technological complexity, with all its downsides, including cost mark-up, and a higher risk for operational failure and breakdown.

Most imaging systems configured for tomography, such as CT scanners of up to generation IV, interventional imagers such as C-, and U-arms, and others, are rotational. This adds costs, caused by complex mechanical arrangements to enable such rotational imaging. Some health systems struggle with budget constraints and increasing demands. At least in part because of costs, at some places, there are no such rotational imaging equipment available.

### SUMMARY OF THE INVENTION

There may therefore be a need for more cost-effective solutions in relation to tomographic imaging.

An object of the present invention is achieved by the subject matter of the independent claims where further embodiments are incorporated in the dependent claims. It should be noted that the following described aspect of the invention equally applies to the imaging arrangement including such a system, the wearable for use with such a system, to the related method, to the computer program element and to the computer readable medium.

According to a first aspect of the invention there is provided a system for facilitating, in embodiments even enabling, tomographic imaging with a non-rotational radiography imaging apparatus, comprising:
input interface for receiving, when the system is in use and whilst there is relative motion between patient to be imaged and a detector or X-ray source of the imaging apparatus, i) 3D position measurement readings acquired by an optical fiber-based system via a set of optical-fiber based sensors arranged at i.1) the patient (PAT) or i.2) at detector and/or source, wherein the measurement readings are relatable to poses assumable by the patient during motion relative to the detector or to poses of the source or detector relative to patient, and ii) projection imagery acquirable by the imaging apparatus of the patient during, or before and after, such relative motion;
a projection direction determiner configured to determine projection direction data for the projection imagery based on the 3D position measurement readings, and
an output interface for providing, for reconstruction, the projection imagery associated with the projection direction data.

In embodiments the system comprises a reconstructor configured to implement a tomographic reconstruction algorithm to reconstruct a tomographic image of a region of interest of the patient, based on the projection imagery and the projection direction data associated therewith. In embodiments, the tomographic reconstruction algorithm includes a tomosynthetic reconstruction algorithm.

In embodiments, the set of optical-fiber based sensors is integrated at least in part in a wearable (WB) worn by the patient during imaging.

In embodiments, the wearable further includes the detector of the imaging apparatus.

In embodiments, the 3D position measurement readings are converted by the optical fiber-based system from shape measurements representative of shape changes experienced during patient motion, or during motion of source or detector, by a length of optical fiber long which the sensors are formed.

In embodiment, the said relative patient motion is self-induced (no device is used in the motion) by patient on instruction, or is induced by a rotational facilitator on or at which the patient resides during imaging, or is induced by at least the X-ray source or the detector of the imaging apparatus being moved past the patient.

In embodiments, the said X-ray source is tiltable so as to maintain alignment with the detector whilst the X-ray source is moved past the patient.

In embodiments, the rotational facilitator includes any one of: a swivel stool, a patient bed.

In embodiments, there is at least a part of the set of fiber-based sensors arranged in a spatial configuration that is essentially maintained during patent motion.

In embodiments, the projection direction data are determined based on a patient pose normal vector, relative to a normal vector of a surface of the detector or a normal vector at the source, such as normal of a surface of the source's housing.

In embodiments, the patient pose normal vector is determined as a normal of a plane defined by three or more reference 3D position readings from among the 3D position measurement readings.

In embodiments, there are three or more reference 3D position readings supplied by reference sensors of the optical-fiber based sensors, arranged to form a polygon at the patient in relation to the region of interest to be imaged or form such a polygon at the detector or the source. Again, the polygon may be laid out at detector's surface or a surface of the source's housing, or in another opportune and pre-defined way. A circular or elliptic or other planar layout may also be envisaged herein.

In embodiments, the reference sensors are arranged to form a triangle on patient's torso.

In embodiments, the optical-fiber based system includes a second set of sensors arranged at the detector.

In embodiments, the optical-fiber based system includes a second or third set of sensors arranged at an X-ray source of the imaging apparatus.

In embodiments, the set of optical-fiber sensor at the detector is run to extend to the X-ray source, the set thus connecting the detector and the X-ray source.

In embodiments, the imaging apparatus is of the mobile type with its X-ray source arranged on a wheeled or tracked frame, wherein the imaging apparatus including its X-ray source is movable past the patient on its frame during imaging.

In embodiments, if the relative motion is not self-induced, the system may further include an immobilization device, capable to essentially immobilize at least an anatomy of the patient during imaging.

In embodiments, the immobilization device includes a mattress or cushion formed from viscoelastic foam to receive therein at least the said anatomy of the patient.

In embodiments, the system may include a motion guidance system to guide patient in their motion when self-induced.

In another aspect there is provided an imaging arrangement including the imaging apparatus and further including at least the set of optical-fiber based sensor at the detector and/or at the X-ray source, for use with a system as per any one of the preceding embodiments, and/or further including the optical fiber-based system.

In embodiments, the imaging arrangement further includes at least the set of optical-fiber sensors at the patient.

In embodiments, the imaging arrangement is further to include the system of any of the preceding embodiments.

In yet another aspect there is provided a wearable for a patient including a set of optical fiber sensors for use in tomographic imaging with a non-rotational radiography imaging apparatus.

In another aspect there is provided a computer-implemented method for facilitating/enabling tomographic imaging with a non-rotational radiography imaging apparatus; comprising:
receiving, when the system is in use and whilst there is relative motion between patient to be imaged and a detector or X-ray source of the imaging apparatus, i) 3D position measurement readings acquired by an optical fiber-based system via a set of optical-fiber based sensors arranged at i.1) the patient or i.2) at detector and/or source, wherein the measurement readings are relatable to poses assumable by the patient during motion relative to the detector or to poses of the source or detector relative to patient, and ii) projection imagery acquirable by the imaging apparatus of the patient during, or before and after, such relative motion;
determining projection direction data for the projection imagery based on the 3D position measurement readings, and
providing, for reconstruction, the projection imagery associated with the projection direction data.

In another aspect there is provided a computer program element, which, when being executed by at least one processing unit, is adapted to cause the processing unit to perform the method.

In another aspect there is provided at least one computer readable medium having stored thereon the program element.

What is proposed herein is an enabler system and method for tomographic imaging that can be retro-fitted to existing radiography systems to re-purpose same for use in tomographic imaging instead of or in addition to a classical projection-based-only radiographic imaging. It includes a set of optical fibers which are connected to a data processing system that converts shape or curvature measurements captured during motion of the patient or equipment, and to convert same into 3D positional data which can then be translated by a software or hardware-based system into directional information that relates to the relative rotation/motion of the patient during image acquisition. The data pair of projection image frames and the projection direction data can then be used for tomographic purposes as envisaged herein.

The proposed system or method facilitates, or, more specifically, enables, performing tomosynthesis or tomography using merely a standard radiography system (as such, incapable of C-arm like movement). Through the optical fiber-based 3D position and orientation determination of the relevant patient's body part or patient body as a whole, tomographic imaging can be done. In some examples, patient can be instructed to rotate the body part while the angle of rotation is recorded with each X-ray image acquired. Alternatively, in situations where the patient's movement would change bone alignment (i.e., joint movements) or would otherwise be undesirable, the whole patient is moved via a platform without changing patient anatomical alignment. A rotating chair or bed might provide such a patient movement. No motor is needed but can be used. The projection images can be interpolated to generate images corresponding to a regular angular interval or can be directly used for tomography, such as in tomosynthesis. The relative motion may not necessarily be one of rotation but may be instead a lineal translation. In some embodiment, it is the patient who is moving. Alternatively, or additionally, it is the imager's X-ray source ("source") or the detector that is being moved past the patient, whilst patient may remain stationary.

The system may thus include in some embodiments: i) an optical fiber-based sensing system in which, ii) the position of relevant anatomical regions of the patient's body (curvature points) and, optionally, of a reference frame attached to the detector can be determined, and iii) positional deviation of the patient can be detected and measured.

The proposed system and method allow i) tomosynthesis/tomography using standard radiograph system, ii) retro-fitting to extant radiography systems with no hardware change for tomosynthesis/tomography, iii) providing an alternative mechanism to perform tomosynthesis/ tomosynthesis/tomography.
*"patient"* is the object of the imaging. Reference to "patient" is not necessarily a reference to patient as whole, but may be a reference to a part, such as body part, anatomical feature, organ, group of organs, body region, and the like. Thus, an object of the imaging may relate to such a part, referred to herein as the region of interest (ROI). Patient as used herein mainly for human patients, but animals, such as pets, in veterinary applications, are not excluded herein.
*"user"* relates to a person, such as medical personnel or other, operating the imaging apparatus or overseeing the imaging procedure. In other words, the user is in general not the patient.
*"pose*" is used interchangeably herein and relates to position and orientation of a body part, organs or of anatomical feature more generally, or to position and orientation of equipment or parts, such as X-ray source, detector etc., relative to a coordinate frame, such as "world"-coordinate system, a specific, agreed location in the exam room, for example.

### BRIEF DESCRIPTION OF THE DRAWINGS

Exemplary embodiments of the invention will now be described with reference to the following drawings, which, unless stated otherwise, are not to scale, wherein:
Fig. 1 shows a schematic block diagram of a radiography system used for tomographic imaging as envisaged herein in embodiments;
Fig. 1A shows a rotational imaging system;
Fig. 2 is an illustration of tomographic imaging based on relative motion between patient and X-ray source;
Fig. 3 are perspective views on patient induced motion for tomographic imaging as envisaged herein in embodiments;
Fig. 4 is a schematic block diagram of an optical fiber-based 3D position measurement system;
Fig. 5 shows an optical fiber system arranged at the patient and a detector as envisaged herein in some embodiments for tomographic imaging based on patient induced motion;
Fig. 6 shows a fiber system arranged at the patient or in a wearable as used in some tomographic imaging embodiments envisage herein;
Fig. 7 is a schematic block diagram of an enabler system for tomographic imaging as may be used in embodiments;
Figs. 8, 9 are schematic block diagrams for optical fiber-based tomographic imaging arrangements in accord with further embodiments envisaged herein;
Fig. 10 is a schematic drawing of an immobilizer device as may be used herein in embodiments for tomographic imaging; and
Fig. 11 shows a flow chart of a method of enabling tomographic imaging by using a radiography imaging apparatus.

### DETAILED DESCRIPTION OF EMBODIMENTS

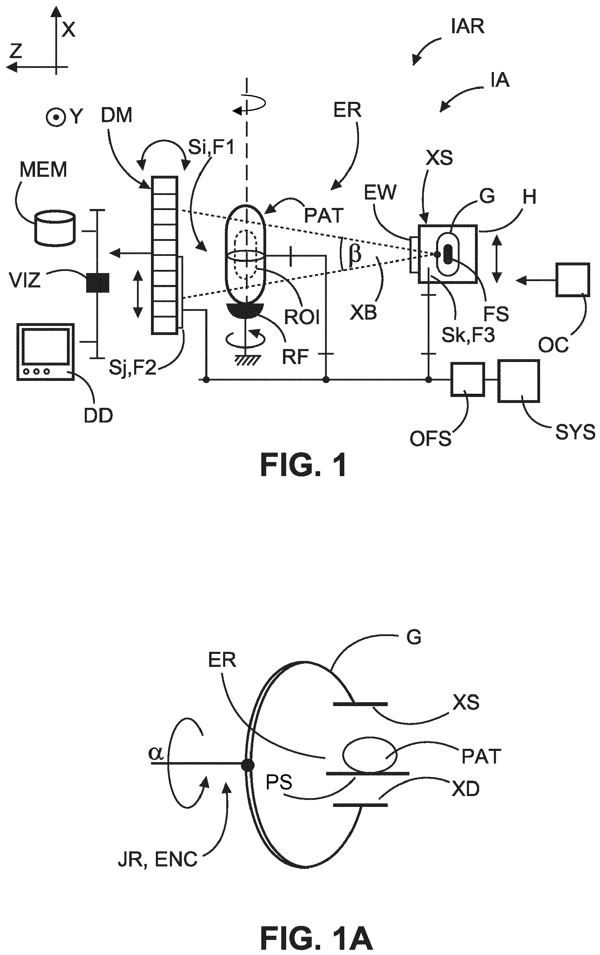

Referring first to the block diagram in Fig. 1, this shows components of a medical imaging arrangement IAR envisaged herein in embodiments.

The arrangement IAR preferably includes an imaging apparatus IA, such as an X-ray based imaging apparatus, preferably radiography imager configured as such for projection imaging. The imager IA is operable to acquire medical imagery of a patient PAT during an imaging session to aid, for example, therapy and/or diagnosis. An example of this is chest X-ray imaging as schematically shown in Fig. 1, but imaging for other body parts or other purposes are also envisaged herein.

The imaging arrangement IAR includes a computing system SYS implemented by one or more fixed or mobile computing devices or systems. The computing system SYS is operable to process projection imagery as provided by imager IA. Broadly, the computing system SYS acts herein as an enabler system that allows turning the, as such, projection-based-only imager IA, into one with tomographic faculties. The system SYS is retro-fittable to existing radiography imagers IA of different types. This will be explained in more detail below, after providing first more context around medical imaging, in order to better elucidate explanations of enabler system SYS.

Generally, the imaging arrangement IAR may be set up in an examination room in a medical facility (hospital), or may be used in outside settings such as by ambulance crews, or on other medical rescue missions, catastrophe relieve operations, etc.

The medical imaging arrangement IAR includes an X-ray-based imaging apparatus IA ("imager"), with an X-ray source XS (such as an X-ray tube), and an X-ray sensitive detector module DM (for brevity, sometimes referred to herein simply as the "detector"). During imaging, the X-ray source XS is energized to produce an X-ray beam XB that issues forth from a focal spot FS and through an egress window EW of detector housing HS, to interact with patient tissue PAT as it passes through patent PAT as he or she resides in an examination region ER between source XS and detector DM. The beam emerges at the far side of the patient and impinges on an X-ray sensitive surface of detector DM comprised of pixels. The detector may be of any type and sort, such as flat-panel detector where the detector pixels are arranged in a matrix layout in rows and columns, to define the X-ray sensitive surface as one in 2D.

The radiation beam XB is modified in its interaction with tissue. The so modified beam is then detected as intensities at the detector pixels of detector module. Some of the detected intensities vary spatially across different detector pixels, the detected intensities thus representing a projection image of internal anatomies, tissues, etc. The intensities are converted by A/D conversion into digital images, which may then be stored on an image memory MEM or may be displayed on a display device DD after visualization through a visualizer VIZ. The visualizer may form of graphics display based on the projection imagery, or such imagery may otherwise be processed as required by the medical task at hand.

The imaging apparatus IA is preferably, as illustrated in Fig 1, of the radiography type, so it is solely based on projection imagery as described. The radiography apparatus IA may be of the fixed type such as mounted in an examination room. For example, the X-ray source may be wall, ceiling, or floor mounted, in a fixed frame structure. The mounting may allow some alignment options such as panning, or tilting as illustrated by angle β. This allows aligning of beam XB with the region of interest ("ROI") of patient to be imaged. The field-of-view ("FOV") may be adjusted in this manner. Likewise, the detector module DM may be ceiling, floor or wall mounted, as desired in a similar frame structure, a stand, etc. The so mounted detector DM may also allow some form of positional orientation modification as indicated by the arrows in Fig. 1, such panning in x and/or y direction, some tilting, etc. In Fig. 1, and herein, directions orientation, position, pose, etc. of imager IA components may be with reference to an imaging coordinate system (x, y, z). Axis z may in general indicate the imaging axis, that is, the propagation direction of the main or central X-ray beam XB.

Such a fixed type imaging arrangement IA as described, may be used for chest X-ray for example, where the patient is generally asked to stand in the examination region during imaging. There may be suitable floor marking to assist the patient in finding the right general location in the room, between source XS and detector DM.

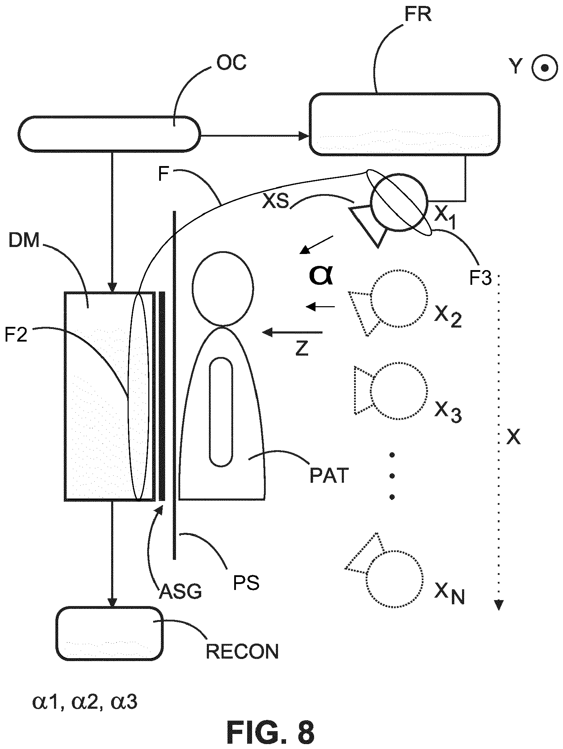

As an alternative to the room-bound radiography imaging apparatus IA of fixed type as per above to similar, the imager IA may be instead configured as of the mobile type. In such mobile type imagers IA, at least the X-ray source is mounted in a mobile frame, on a dolly or other tracked or wheeled, mobile, frame structure FR (see Fig. 8, to be discussed in more detail below). Such mobile imager IA can be moved by staff freely across a room, or from one room to another for more flexibility when imaging patients, for example those who are too infirm to make their way into the exam room. In some such mobile imagers IA, the detector module DM may afford free and untethered handling, with no physical attachment, other than possibly a cable for image data transfer, but even this may be replaced by a wireless image data transmitter. Such detector module DM may be generally a square or rectangular shaped plate (but can be of any form factor), which can be positioned behind the patient. For example, such type of detector modules DM may be used for bed ridden patients that are too infirm for sitting or standing. The untethered detector module DM is placed for use behind the patient, essentially to become sandwiched between patient and bed top when in use. The generally wheeled frame structure RF (again see Fig. 8) is positioned by user, with source XS aligned as described above for the fixed-type imager, and projection imagery may then be acquired, again, as substantially described above for the fixed-type imager.

The imaging arrangement IAR using an imager IA of fixed or mobile type may include a patient support PS situated in examination region ER. Such a patient support PS may include a table structure, couch etc., on which the patient may lie during imaging, or on which patent may position the ROI, such as an extremity, etc, for musculoskeletal imaging. The configuration of the imaging arrangement is such that the ROI/patient resides on the support PS, and between the source XS and the detector module DM.

Were it not for the proposed imaging enabler system SYS (which will be discussed momentarily in more detail), the radiography imager IA of any of the two types (fixed or mobile) as such, is only capable for projection imaging. This is in distinction to other imaging systems, such as illustrated in Fig. 1A, which are specifically configured for rotational tomographic imaging. In such systems, the X-ray source SS and detector DT are rigidly connected in gantry G. Gantry is a journalled JR to allow rotation of source SS and detector DT around the examination region, with the ROI/patient in it, as schematically indicated in Fig. 1A. This allows acquiring projection imagery from multiple directions, which imagery can be combined by a tomographic reconstruction algorithm to reveal sectional or 3D volume imagery of the ROI. Such reconstructed imagery is different from projection imagery which is part of projection domain (detector), whilst reconstructed imagery is situated in image domain, part of 3D space in the examination region ER. Such tomographic rotational equipment as illustrated in Fig. 1A is more expensive in terms of parts, maintenance, and is more prone to glitches and faults, as compared to the simpler radiography setup IA of Fig. 1, with fewer (or at times no) moving parts. For example, the rotational tomographic system, such as a CT scanner or C-arm imager in Fig. 1A, requires the expensive gantry G set-up that holds the X-ray source SS and the detector DT, slip ring arrangements to enable data transfer, the said journaling JR with complex and high-cost ball-bearing arrangements to allow for smooth rotation during imaging, etc. In addition, tracked rotation is required in such systems designed for tomographic imaging, which calls for complex electro-mechanical rotational encoder components ENC. Such encoder components ENC track the gantry G's rotational motion by recording associated angles. The recorded angles are then associated with the imagery acquired at different such angular directions, before the acquired projection data and their recorded angles are passed on for reconstruction.

Thus, the non-rotational, radiography system in Fig. 1 as envisaged herein, is in distinction to the rotational systems in Fig. 1A, the latter designed and intended for tomographic imaging from the outset. Alas, the radiography imager IA as envisaged herein in embodiments is re-purposed for tomographic imaging, without requiring any of the above mentioned high-cost and fault prone parts, such as journalling JR, encoder EC, slip rings, rotatable gantry with source and detector, etc. Instead, the largely software-based enabler system SYS is provided herein, that enables the radiography system of the likes of Fig. 1 (and cognates thereof) for tomographic imaging.

Broadly, in terms of hardware components, the enabler system SYS, includes or cooperates with a 3D position measurement system OFS, preferably optical fiber based, that collects such measurements in relation to the patient PAT during imaging. Broadly, the enabler system SYS processes the 3D position measurements system provided by system OFS, to enable tomographic imaging. Thus, even existing radiography systems of the fixed type or mobile type, originally configured only for projection-imaging (Fig. 1), can be retro-fitted by the proposed enabler system SYS to so repurpose such radiography imagers IA for tomographic imaging in a cost effective and flexible manner. The system may be at least partly installed in the imager's IA operating console OC, or indeed in any computing device, such a smart-phone, tablet, laptop, or their "sedentary brethren", such as a desktop computer, workstation, etc.

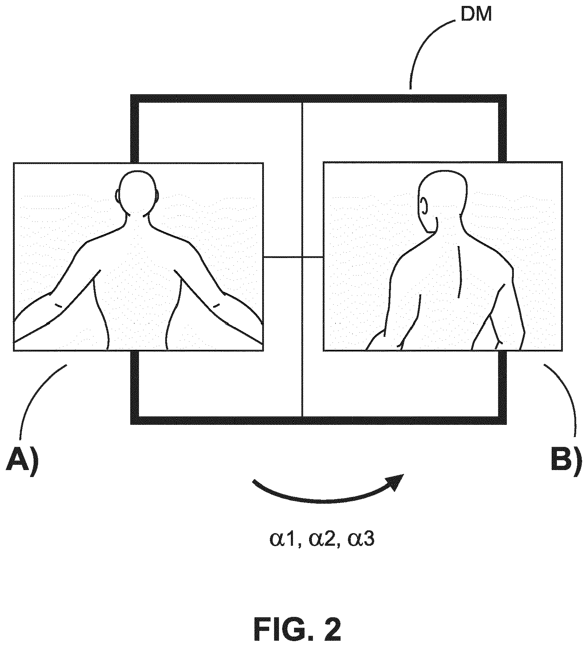

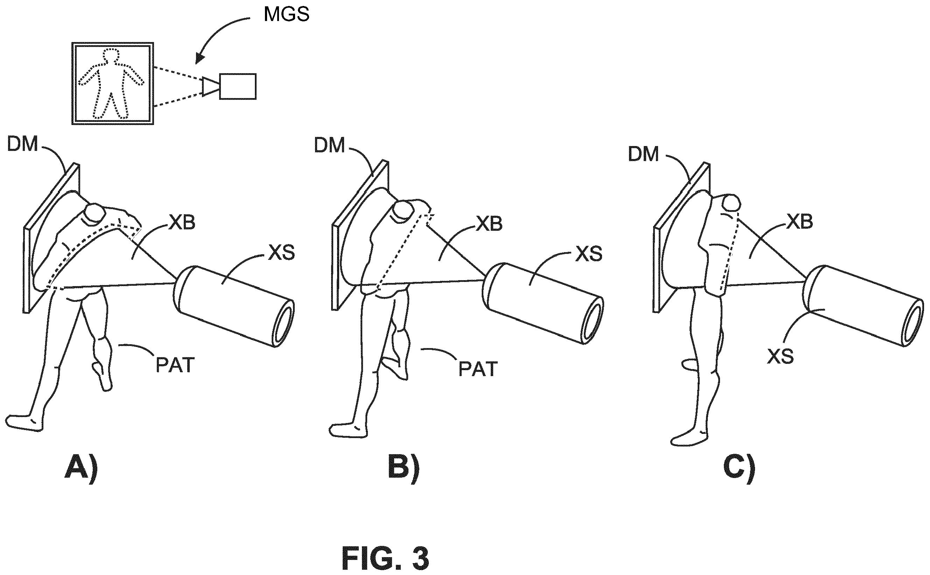

The proposed tomography enabler SYS for use in radiography set-ups IA is conceptually illustrated in Figs. 2,3, to which reference is now made.

Referring first to Fig. 2, and with continued reference to Fig. 1, the 3D position measurement system OFS uses an arrangement/system of optical fibers, schematically shown in Figure 1 as sensors Sj, along respective fibers Fj, (j =1,2.). Three such optical fibers F1-3 are shown, one for detector, source XS and patient PAT, respectively, but one such set of sensors S 1 in a single or more fibers F1 arranged merely at the patient may be sufficient in some embodiments. When reference is made herein to such sensor(s)/ fiber(s) generically as used by 3D position measurement system OFS, reference symbols "F", "S" are used herein.

In the proposed imaging set-up IAR, it is the patient PAT that undergoes rotational movement, either self-induced or facilitated by a rotational facilitator RF. In the self-induced or self-propelled case, the patent may stand for example in the examination region, rotates about a rotation axis (see the dashed line extending along direction X in Fig. 1), substantially coincide with patient's longitudinal (cranio-caudal) axis. This rotational movement by patient is against stationary source XS and stationary detector DM, as illustrated in perspective views in Figs. 2A, B and Fig. 3. Instead of being self-induced, such patient motion may be facilitated by a technical device that is the rotational facilitator RF, such as a swivel chair, or some such.

During the rotational patient motion, optical fiber-based system collects 3D positioning information through the fiber F1 arranged at patient. Whilst patient is changing their pose, a stream of shape measurements is generated by fiber F 1, in response to shape changes of the fiber F1, caused by patient's rotational or other movements. The stream of shape measurements is passed on from fiber F 1 to a data processing system unit SSP of the 3D position measurement system OFS. Data processing system unit SSP computes from the stream of (shape) measurements, 3D position information in 3D space for certain points on the part of the fiber F1 arranged at the patient. The system SYS may translate the 3D position measurement into directional, angular data, as shown in Fig. 2, with projection direction data α1, α2, α3 corresponding to different poses of patient PAT. The said patent poses corresponding to two instances of the projection direction data α1, α 2, α3 are illustrated in perspective drawings A), B) of Fig. 2, with patient PAT residing in front of the detector module DM. The view afforded in Fig. 2 is that along the imaging axis Z extending into the drawing plane in Fig. 2 and towards detector DM's plane.

Either self-propelled or facilitated, whilst patient PAT rotates around their longitudinal axis (dashed line in Fig. 1), the stationery X-ray source XS is energized and the detector DM operative to acquire a series of projection images λi=1-3. Some or each projection image λi is associated with the respective projection direction data α1, α 2, α3, as was recorded by the optical fiber based (3D) position measurement system OFS at the respective time the respective projection image was acquired. Thus, each projection image ("frame") λj is associated with the respective directional angle αj. In this manner, a dual data stream λj, α is generated. The so associated angularly tracked data λj, α thus allows using any existing tomographic algorithm to reconstruct therefrom volumetric or sectional imagery V for storing, viewing or other processing, as required. Of note, such angular tracking does not require expensive, fault-prone and maintenance intensive encoder ENCs as does the rotational setup CT or C-arm setup Fig. 1A. Such projection frame λj vs angle association α can be achieved by using time stamps/tags t awarded by imager and position measurement system OFS for their respective measurements, for example.

In general, patient PAT can be expected to carry out a limited angular rotation in most cases, in which case tomosynthesis reconstruction algorithm may be more appropriate. However, nothing herein precludes patient PAT to perform a rotation of at least 180⁰, even 360° or more, to so allow computing a full tomographic 3D volume, using any one of a range of standard reconstruction algorithms, such as FBP (filtered-back projection), algebraic, iterative, etc.

Fig. 3 shows the same concept as in Fig. 2, but in a perspective view, partly from above, whilst patient PAT undergoes rotation (self-induced or facilitated) thereby assuming different poses as indicated in the sequence of Figs. 3A, B and C.

Patient PAT may be guided in their rotational motion by a motion guidance system MGS to encourage performing correct, that is, uniform rotation around their longitudinal axis as accurately as possible, preferably with rotation axis maintained throughout rotation parallel to detector DM's image plane.

The motion guidance system MGS may be configured to provide vocal or audio cues through a speaker system. In addition, or instead, motion guidance systems MGS may be video-assisted as is preferably envisaged herein. For example, a video stream may be displayed on a display device. The video information may represent an outline of a moving human figure which the user is encouraged to follow. A shadow play may be envisaged with a full-size screen on which a silhouette of the intended movement video is projected onto. A projector video source behind the patient may cause a shadow of patient being cast on the screen, together with the silhouette outline of the intended motion. Patient is then encouraged to follow the guidance, so that their shadow conforms to that outlined silhouette in the projection video. However, a video stream displayed on whichever display device as computer graphics for example, may be used instead or in additions. For example, a virtual avatar is moving on the computer display which shows the optimal movement trajectory and deviations of it. Many variations of the motion guidance systems MGS are envisaged herein in embodiments, so long as they are configured to guide patient in following a target rotational motion.

As mentioned, the rotational movement of the patient during imaging may not necessarily be entirely self-induced but may be facilitated. As shown in Fig. 1, the patient may be sitting instead on a swivel chair or stool RF or other rotational facilitator RF, and it is the chair or stool that rotates, either motorized or, more preferably, under inertia after an initial force input, such a push off from ground, nudge, etc., conferred by user or patients themselves. Another option is to have a rotational platform inserted into the floor of the examination region, preferrable flush therewith. The, for example circular platform, may be either propelled by a motor, or, preferred herein, by patient or medical user, using again, inertia after an initial impulse input. Once the platform is in motion, imaging can commence, to acquire the angularly tracked projection data, as described earlier. When using such rotational facilitator R, be it a stool, chair, or the floor inserted platform, the patient may assume any desired base posture, such as sitting upright, standing, squatting, as required, and imaging continues whilst the patient thereon rotates around its axis due to inertia or other. The movable platform in the flooring may require substantial outlay which is less preferred herein for an easily retro-fittable tomographic imaging enabler system SYS, OFS such as envisaged herein. Specifically, a simple swivel stool or chair with or without headrest, backrest or armrest can be purchased with minimal outlay from any office furnishing outfit and may be sufficient for present purposes.

Thus, in its basic form, the proposed retro-fittable tomographic enabler system includes the described projection direction data processing system that may be run on a computer such as a laptop computer or desktop computer, or indeed on any computing device, handheld or not. In addition, interfacing with the optical fiber-based 3D position measurement system OFS is preferable, as will be described in more detail below. Interfacing may be wireless or wired, as required. The reconstruction computations may be run locally on the system SYS, but may also be outsourced or off-loaded, for example to a server, workstation, etc. The system may thus be arranged in benefit in client-server or Cloud infrastructure. The local part of the system at the imager IA may thus be merely concerned with coordinating collection of data λjα, whilst reconstruction may be done elsewhere, such as on a more powerful server computer, for example. The data λjα is dispatched to such server or other computing system, workstation etc. that implements the reconstruction algorithm. Upon reconstruction, reconstructed image V may then be returned for local viewing, processing, storing, control purposes, etc.

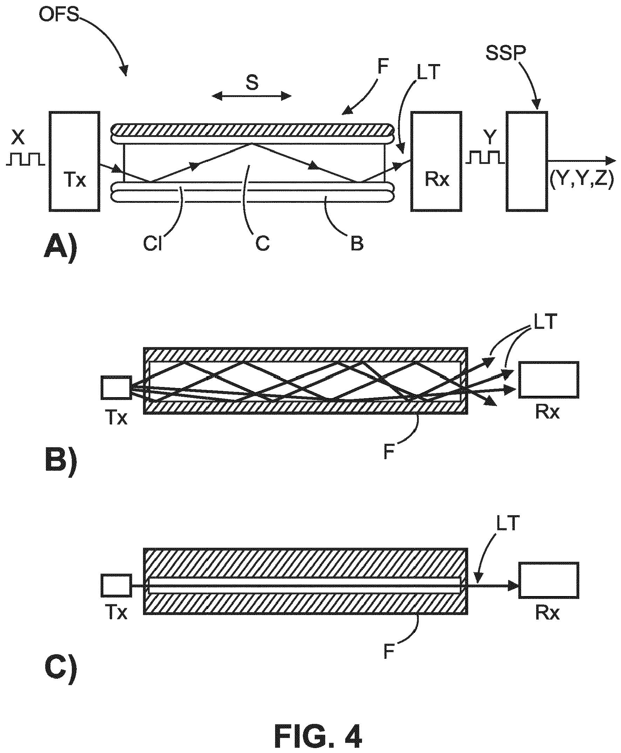

At Fig. 4 to which reference is now made, the 3D position measurement system OFS is described in more detail in various embodiments.

Broadly and preferably, optical shape sensing is preferred herein for the optical fiber based 3D positional measurement system OFS, but other non-invasive and non-nonionizing shape sensing principles and technologies are not excluded herein.

The 3D positional measurement system OFS includes in embodiments a shape sensing device SSD, that includes a shape sensing processing unit SSP, and plural sensors S, mentioned above. The sensors S are preferably defined in or on, but in either case along, a deformable elongated component of the sensor device, such as a probe, arm or the like. The shape sensing device is preferably based on optical light in which case the said probe or arm may be implemented as a length of the mentioned one or more optical fibers F,F1-3 as mentioned earlier. Preferably, multiple such fibers may be used that each define a separate set of sensors. Preferably at least two, more than two or all fibers are coupled to the same shape sensing processing unit SSP, and their shape sensing readings are processed together to produce the output result. The fiber F may have circular, elliptic or other such cross-section, or may be instead arranged flat, as a band, strip, or tape for example. Suitable such optical fibers are described in Applicant's US 2009/0137952.

One way poste of patient PAT, detector MD, or source XS may be measured is by arranging a first set sensors Si (also referred to herein as "patient sensors") of the shape measurement device SSD in relation to the patient, in particular in spatial relation to the region of interest.

Optionally, a second set of such sensor Sj (referred to herein also as "auxiliary sensors") is arranged in spatial relation to the detector module DM. The auxiliary sensors in respect of detector module may be arranged at or on the mobile DM or may be integrated therein. However, it may be sufficient for the sensors to be arranged in a prior known spatial relationship, not necessarily at the detector, but elsewhere in the examination room or at the other parts of the X-ray imager IA, arrangement of the second set of sensors Sj. In the following, a reference to the/a *"fiber F"* may be taken in addition as a reference to the respective set of sensors that makes up at least a part of that fiber F.

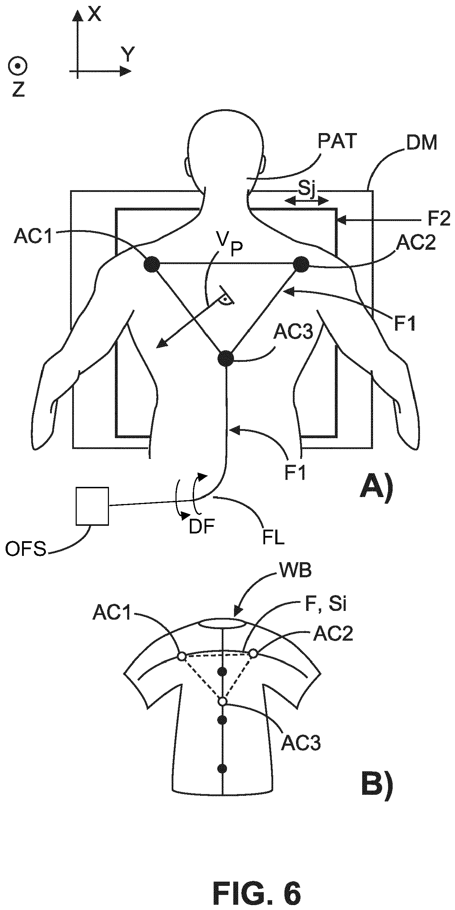

During pose changes, a feeder line FL part of the fiber F, F1-3 that runs from patient, source or detector where the plane is defined by fiber layout as mentioned above, to data input interface of shape sensing processing unit SSP experiences deformation DF (see Fig. 6 A), where this is illustrated).

This deformation causes light that travels through the optical fiber to be internally reflected in a particular reflection pattern that correlates with deformation of the fiber F. Thus, the reflection pattern can be correlated to curvature that the fiber is experiencing along its length. As each sensor or point along the length of fiber experience its own location curvature, a corresponding set of curvature values (one for each senor) may be provided as output.

The shape sensing processor may compute from the curvature values, 2D or preferably 3D point coordinates as the output result. However, such conversion is not necessarily required, and output may be provided as curvature data instead. In fact, the format or nature of the output data is immaterial herein, so long as the output data is indicative to 3D position of points along fiber F in 3D space relative to the world-coordinate system (X;Y;Z). The output data may relate to stress or strain measurements. Basic operation of the above-described optical shape is illustrated in block diagram of Fig. 4A).

An input signal X controls a light transmitter TX to transmit the light LT through fiber F. A cross section of core of such an optical fiber is shown, and it will be understood that there may be more than one such cores wound, braided or bundled to otherwise combined so make up the fiber. A cross section of the fiber is shown, with a core C and optional cladding zone buffers in which the core is ensheathed. Light ray travelling through fiber F undergoes (internal) total reflections and is received at light detector RX to produce an output measurement signal that represents the reflection pattern experienced by the ray and correlatable with shape of the fiber, and hence body posture due to attachment of fiber to relevant body part as described above. The reflection patten may be processed by processor SSP into the said indicative output signal. The processor SSP may include a solver to solve, based on the shape measurements (stress, strain or curvature values), a set of Frenet-Serret equations to obtained output data indicative of shape and/or 3D positions that represent posture.

Similar readings may be received from optional second fiber F2 (for example coupled to detector module) to provide a second stream of shape sensing readings which can be co-processed with stream of shape readings from the first fiber F 1, to more robustly compute posture relative to detector module DM. Using a single or more fibers arranged solely at the patient may also be possible, in which case no fiber(s) F2 need be arranged in relation to the detector module DM.

It will be understood that in some embodiments, the sensors Si,j may be discretely arranged as a set of Fiber-Bragg-Grating (FBG) sensors, although this is not necessarily required in all embodiments as other technologies are also envisaged. For example, the sensors may include point positions or locations along the fiber, so are not necessarily discrete components/structures as in FBG or similar, but are rather quasi-continuous lineal arrangements of point locations along core C of the fiber.

In other arrangements, the FBG per core are not necessarily spread out evenly over the core's length, but are arranged instead towards an end portion of the respective core, or elsewhere along the respective length of the respective core. There are preferably plural fiber cores per fiber F, such as 2 or 3 or more. 3 cores is sufficient for good results in 3D, but 2 may suffice in some circumstances. Thus, a reference herein to a set of sensors S, may be construed as a reference to the respective multiple cores of the fiber F and/or to multiple FBGs in a core, or to other arrangements in or at the fiber core(s) that enable measuring shape.

Specifically, light transmitted through an optical fiber may be used to estimate the 3D curvature of the plane where the optical fiber is placed. In this process, different wavelengths of light are transmitted through the optical fiber and the phase difference and Time of Arrival (TOA) is calculated. From these phase differences and TOAs, approximation of the curvature points of the plane is derived. Alternatively, the mentioned FBG-based sensors are used. Each point on the curvature may be defined by a tuple (x, *y, z),* representing the positional vector in the 3D space. Sending the different wavelengths may be done sequentially in single mode (Fig. 4C) where a single light ray at a given wavelength is transmitted at a time, or by using multi-mode fiber optics (Fig. 4B) where lights at different wavelengths are send down the fiber F at the same time.

Thus, and preferably, shape sensing processing unit SSP is configured to that fiber F can be interrogated for shape/curvature, and thus 3D point coordinates, for any point along its length, on particular to reveal the respective 3D position coordinates of the 3 points AC1-3 at patient, or at detector DM, or source XS.

The end portion of fiber F that terminates at the patient, source or detector is the distal end, whereas the other end portion, the proximal end, of fiber F (which is the end portion in which the feeder line part of fiber terminates) feeds at interface into shape sensing processing unit SSP, such as via a suitable optical cable socket, plug, etc. Each F F1-3 may comprise a system of multiple, possibly interconnected, fibers.

In general, in the sensing operation, points of references may include inflection points on the fiber core. The shape sensing mechanism in general monitors the respective angle formed at the inflection points. If a second set of sensors at the detector is used. as is preferred herein, alignment of the angles with the reference frame is monitored. The reference frame is defined by the second set of sensor Sj at the detector, in addition to the set of sensors Si at the patient.

It will be understood that the embodiments of the shape sensing device SSD in Fig. 4A-C are merely examples and other arrangements are also envisaged herein so long as shape measurements are obtainable that are correlatable to posture changes, substantially as described herein. Optical light or infrared light is used herein, but other frequencies in the non-ionizing range are also envisaged herein.

In sum, the signal processing system SSP may thus be able to convert the shape and/or curvature measurements of the fiber(s) F1 (or if use, F2, F3) into 3D co-ordinates of points in 3D space (*X,Y,Z*)*,* relative to a world co-ordinate system. In particular, the 3D point position information may be taken relative to an arbitrary reference point in the examination room. The optical fiber-based 3D position measurement system OFS may be set up in an initial calibration phase by calibrating its readings relative to a chosen world reference origin of the co-ordinate system.

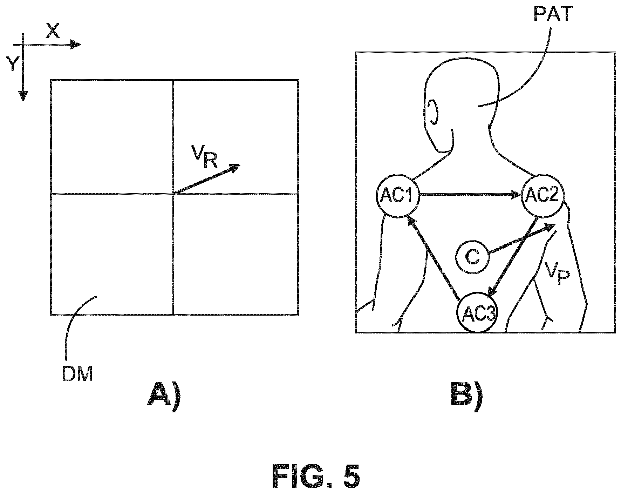

As observed above, operation of the tomographic enabler system SYS, OFS envisages arranging one or more optical fibers F1 at the patient, in particular, in a layout as is shown in Fig. 5B in more detail, to which reference is now made, with continued reference to Fig. 5.

Patient fiber F1 may comprise three strands of fiber, arranged to terminate at their distal ends in endpoints thereof, thus defining three or more anchor points AC1, AC2, AC3 as shown in Fig. 5B. However, any pre-defined points on the fiber F1 (strands) may be used instead. Thus, the term "(optical) fiber" as used herein does not imply it being a single strand of fiber although it may well be in some arrangements, but may form, as shown in Figs. 5,6, multiple strands of fiber, each functional on its own or in combination, and each interrogatable for shape changes/position information along its respective length. Alternatively, indeed, single such fiber (strand) is used, and it is run in a layout to define the three anchor points AC1-3.

Fiber F1 is preferably so arranged at the patient, so that there is no mutual motion or displacement among the three points AC1-3 during patient's rotation. This can be done in one embodiment for example by arranging, as shown in Fig 5A and Fig. 6, the fiber in a suitable geometrical shape that defines the at least 3 anchor points AC1-3. For example, fiber F1 may be run in a triangular layout on back of patient's torso. The three anchor points AC1-3 form thus vertices of the triangle layout, with the distal portion (feeder line part FL of fiber F1) of fiber 1 running off patient's back, along the floor for example, to connect at interface with the processor SSP of 3D position measurement system OFS, to so provide the shape measurements for processing there. For example, the triangle may be formed by the points AC1-2 at shoulder blades, with point AC3 at the lower, lumbar, portion of the spine. Other configurations of the anchor point layout are also envisaged herein, so long as they allow, preferably unique, definition of a plane, the "patient plane", for reasons that will become apparent shortly.

Whilst the mutual constellation of the 3 anchor points does not change, the feeder line portion of the fiber will experience some shape changes during patient' rotation, such as some form of torsion etc. Such shape changes can be translated by processor SSP into a stream of 3 individual 3D position coordinate readings, one such stream for each anchor point AC1-3.

The patient plane defined by the (at least) three anchor points AC1-3 can be computed by analytical geometrical methods. The plane defines a normal *V̅ₚ* of the patient plane and may readily be computed, given the three plane defining points AC1-3.

In a similar vein and in some optional embodiments, a second, similar fiber F2 set of 3D position measurement system OFS may be arranged at the detector DM to define a reference plane in relation to detector DM there. For example, the "detector fiber" F2, as it may be called herein, may be run around the edge of the detector module to define three or more points there (not shown), and thus a reference plane with its own normal vector, the reference vector *V_{R}.*

At suitable time increments during patient motion, the angle between the two normal vectors V_{R}, V_{P} (one at detector plane and the other at patient plane) may change, and may thus reveal the projection directions α1, α2, α3, etc. over time, as described above in Figs. 2 and 3, with associated projection frames acquired to produce the dual angle v frame data stream λj,α. Thus, the projection data may be computed on geometry grounds based on the changing mutual spatial constellation of the two normal vectors, and thus the co-changing angle α between them. The normal may be located at centroids C of the planar layout, such as the triangular (or other) layout as illustrated at Fig .5B. A similar centroid location for the detector normal *̅V̅*̅_{R} may be envisaged herein. Other reference locations for the normals are also feasible herein, and are not confined to centroids.

The reference detector plane is sketched in Fig. 6A. Specifically, Fig. 6A shows a view along the imaging direction z, with patient PAT in front of the detector module. The triangle arrangement of the anchor points A1-A3 is defined by patient fiber F1 and the feeder line part FL of fiber running off the patient's back for example. As alternatives, arrangement of fiber F1 on front (chest) of patient or, around patient's head are also contemplated herein in embodiments. For example, the fiber F1 may be arranged around parts of the front bone and the parietal bone. The fiber F1 may be integrated into a wearable WB, such as in a headband. The three or more anchor points may be placed apart at about 120° around the upper cranial portion of the patient's head. However, this and other fiber F1 layout arrangements at patient may not need to be uniform in all embodiments. Yet another embodiment is shown in Fig. 6B, with the fiber F1 defining the three or more anchor points AC1-AC3, and integrated into another type of wearable WB, such as an upper torso garment, a shirt or hospital gown, etc. This and other wearable WB described herein can be worn by patient during imaging. The fiber F1 is suitably integrated therein, so that it assumes the shown triangular layout or other suitable plane-defining layout, when wearable WB is so worn. Different sizing of wearable WB may need to be provided, given differing patients' heights and physical constitutions. Yet another patient fiber F1 arrangement contemplated herein is around patent's hip, as a belt WB for example.

Alternatively, the anchor points of Fiber F1 may be attached by glue pads to patient's skin, not unlike those used for electrodes in taking an ECG, for example.

In the embodiment where the imager is of the mobile type, the detector module DM may be integrated into such as wearable, such as into the back-covering portion of gown or shirts. For example, the detector module may be sown into the fabric , or may be held in place there by hook-and-loop fasteners (such as Velcro^{™} patches), or similar. The detector may be so integrated with the optical fiber, or instead of the optical fiber.

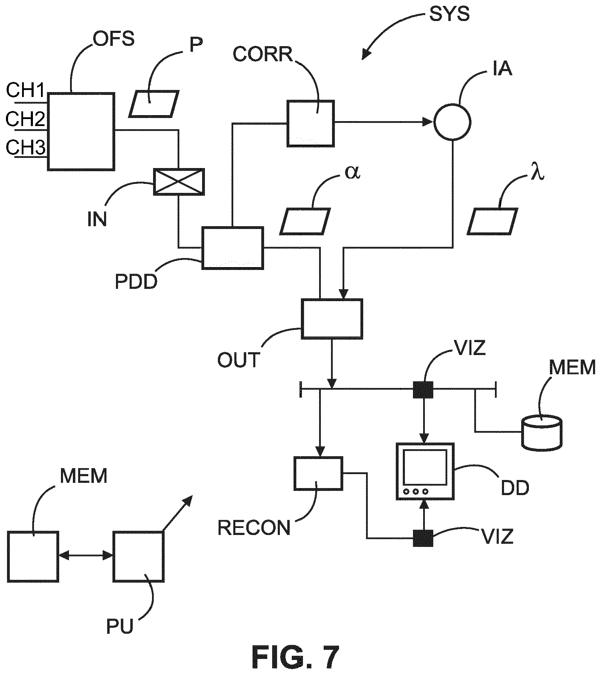

Reference is now made to Fig. 7, which shows a schematic block diagram of the retro-fittable tomographic enabler system SYS as envisaged herein.

The optical fiber-based system OFS is provided for at least one, in embodiments two or three channels CH1-CH3, of shape measurements or curvature measurements, which are transformed into 3D positional information (3D coordinates relative to world-coordinate system) of the anchor points at patient and, optionally, in addition, on detector plane. The 3D positions of points define patient plane and optionally detector plane. Each plane may be defined by a respective set of the three anchor points. The third channel CH3 for a third fiber may be arranged in either embodiment (single fiber F1, or dual fiber setup F1, F2), in addition at the source XS, as will be explained below in more detail.

If the detector DM is stationary at (substantially) all times during imaging (or at least if there is no inclination of detector plane during imaging), the reference normal *V_{R}* of detector plane is a fixed, pre-defined parameter, and hence no detector fiber F2 is needed, and there is only a single channel fiber input from patient fiber F1.

The positional 3D information, comprising 3D co-ordinates P=(X^{AC}i,Y^{AC}i,Z^{AC}i) per channel of the at least three anchor points ACj (anchor points at detector or source are not specifically shown in the Figures), is received at input port IN, and is then resolved by a projection data determiner PDD into the projection direction data α, as explained above. In particular, the projection direction data may be computed at the respective angle α between the two reference vectors V_{P}, V_{R}, and this computation for αj is preferably done at the imaging frame rate. Thus, a stream of projection direction data α=αt is produced during patient rotation and imaging.

The projection direction data α is output at output port OUT. The data α may be associated with a corresponding acquired projection frame λt, acquired during the rotation of the patient. Thus, an associator component at output port associates each or some angle data αt, with corresponding frame λt, to so fashion the dual channel data stream (λt,αt) = λj,α. Time stamping may be used for this association. Projection data αt may be inserted as metadata (header data, etc.) into the corresponding projection frame λt, such as it may be done for DICOM or similar formats. The frame and angular pair data (λt,αt) = λj may be stored in memory MEM of one or more computing systems PU that run system SYS.

Preferably, and as mainly envisaged herein, the associated frame vs angular pair data (λt,αt) = λj is foremost passed on to tomographic reconstructor RECON that uses a tomographic or tomosynthesis algorithm to produce the volumetric sectional slice imagery V. A whole volume may be produced, or merely one more slice images, as required. Tomographic image V can be visualized by suitable rendering software on the display device DD, either in real time during the imaging session, or later during review by the radiologist for example as required and when requested.

Optionally, and in addition, the frame vs angular pair stream data (λt,αt) = λj may be stored in memory MEM for later analysis, statistical analysis, or for educational purposes, and/or may themselves be visualized by the visualizer VIZ on the display device DD.

Any unwanted motion of the imager IA, in particular of its source XS or detector DM during imaging, or incorrect patient rotation may be corrected for by an optional corrector module CORR, based on the precise 3D position measurements as received from the optical fiber-based position measurement system OFS. Operation of corrector may be based in addition on such position/pose measurements as received via the mentioned third/second fiber F3 arranged at the source XS. The fiber F3 may be run between source XS and detector D, or between source and patient. In fact, the third fiber F3 may connect any one or more of patient, source, detector, in any combination. The arrangement is again in a plane-defining manner to obtain a third/second normal vector at "source plane". The distal portion of fiber F3 may be suitably arranged at source XS housing HS for example to define such a plane with at least reference points, similar to the patent of source plane. The corrector operation is based on computing deviation angles between pertinent normal, such as detector DM normal and source XS normal, as explained above, based on which correction can be applied to the data (λt,αt) = λ. In addition, or instead, correction may be used to indicate visually, or via audio to user that there is misalignment. Patient rotation is deemed incorrect herein if rotation velocity is non-uniform, or if patient rotation axis is not parallel to a detector plane. In terms of correct source-detector alignment, this is preferably so that imaging axis z extends from focal spot FS to detector DM plane, and intersects there perpendicularly and preferably at center point of detector's imaging plane. Any misalignment can be picked up and corrected for by corrector CORR, based on computing angle between the detector normal VR and normal at source, similar to what was described above for normals *V_{R}, V_{P}.*

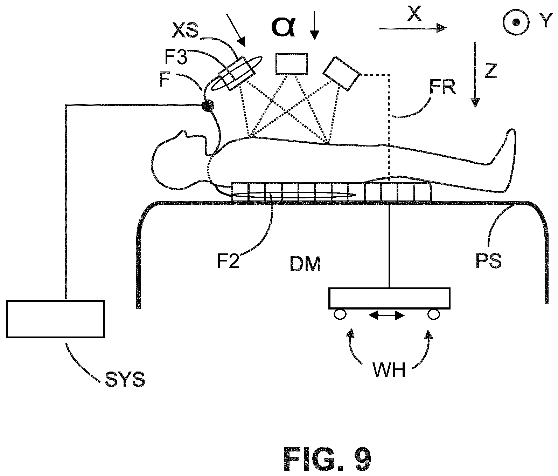

Reference is now made to alternative radiographic imaging arrangement in Figs. 8 and 9, repurposed and enabled herein for tomographic use by enabler system SYS. Such use is again based on similar fiber-based 3D position measurement system OFS as described above. However, the embodiments in Figs. 8,9 are not necessarily reliant on patient induced motion, as such may not always be opportune, for example for the elderly, those in ICUs, or those who have seen surgery and are awakening in recovery room, etc: in short, for all those too infirm for self-propelled physical motion, in particular those confined to prostrate body position (Fig. 9), for whatever reason.

In such situations, it may be useful to have the X-ray source XS move relative to the patient PAT, rather than the patient move relative to source XS, as mainly described above. In such situations, to still enable tomographic imaging with radiography system along the principles described above, the radiography imager IA of the mobile type, described above in Fig. 1, and illustrated in Figs. 8,9 is preferred herein. A frame FR of mobile imager IA on which the source XR is mounted is moved past the patient by staff for example. The scan path may be lineal, along an edge of the bed PS on which patient lies (Fig. 9), or along any other line passed the patient moved. In a chest X-ray (Fig. 9), the frame FR with source XS mounted thereon may be moved past patient. In this embodiment, patient PAT may stand, sit, etc. still in the examination room, This arrangement may be suitable for patients that are unable to move, rotate, due to limited mobility, or be it that they are not expected to follow movement instructions (e.g., children) at the requisite level of compliance and accuracy as may be desirable in situations described earlier on, for example in relation to Fig. 4. The scan path of source XS along positions x=j x1, x2, ..., xN is shown in plan-view for better illustration. At each position xj, a respective projection frame λ is acquired. The scan path may not necessary be a curved around the patient, but could also be lineal, for example for tomosynthesis applications.

In these embodiments, the positional 3D data from which the associated angular data α is computed by system FS is again collected by fiber F, F2, F3, arranged at source XS and detector DM, and at each in a planar-defining-layout as explained above at Fig. 3. Preferably the fiber F extends from source XS to detector DM. This connecting part F allows sensing the angular deviation between the source and detector normals. A single fiber may be used and run as F, F2, F3 between and at source XS and detector DM, or a system of interconnected two or three fibers may be used. A similar arrangement is shown in Fig. 9, with patient in prostrate position on bed PS with detector module DM sandwiched in between patient PAT and bed top, as earlier described. The detector module DM may include an anti-scatter grid AGS in this and other embodiments (such as in the fixed type imager IA in Fig. 1).

The fiber or fibers F, F2, F3 so laid out are all routed via the one or more feeder line parts FL to feed as channels CH1-CH3 into data interface of optical fiber-based 3D position measurement system, with the projection direction determiner PDD of system SYS computing the angular projection direction data α based on the angle between source and detector normal. It is the joint measurements received from the fiber (sections) F, F1, F2 that are preferably used herein to accurately determine the projection angle.

The mutual movement between the tube and the detector module will be registered by the fiber F connecting the two components -the source and the detector module - and this can be translated again into projection direction data. Specifically, it is in particular the interconnecting part F between source XS and detector DM that experiences shape changes during imaging motion between source and detector. From these shape changes, the deviation angle between the two normals (at source and detector), and hence the projection direction can be computed. Having one single fiber run between the two components - the source and detector - is one embodiment, but having two separate fibers F, F2 both feeding as two channels into the OFS system is also envisaged herein.

The angular data α is again associated with the acquired projection frames λ, which are then fed into reconstructor RECON for tomographic reconstruction, as explained above.

In the above examples in Figs. 8, 9, some form of simple motorization may be considered such as tracked, or an electric motor in an at least semi-robotic embodiment, integrated into a dolly, or undercarriage of any kind on which the frame holding the source XS is mounted. The imaging robot may be programmed to proceed along a scan path past the patient, for example, in curved scan path around the patient. However, this may again call for hardware outlay, which may be undesirable. Thus, having instead staff move the source XS past patient by pushing or pulling a wheeled undercarriage supporting the source XS on its frame FR may be all that is needed herein to enable some form of tomographic imaging, be it only tomosynthesis, where only a relatively short scan (lineal or curved) path past the patient need be traced out.

Steering the mobile imager's source XS around ROI in an arc, possibly facilitated by wheels WH with movable rotation axes, as simple as castors/swivel wheels, are also considered herein to enable more tomographic imaging results.

In the above embodiments Figs. 8,9, the X-ray source XS may be angulated so that it maintains alignment with the detector module, although this is optional. Preferably, the angulation and motion are caused manually by clinical user.

As will be appreciated, although the embodiments in Figs. 8, 9 use some form of motion by X-ray source XS, none of the said embodiments Figs. 8, 9 require in particular the complex encoder scheme ENC, journaling JRL and slip rings needed for a tomographic rotational system, as discussed previously in Fig. 1A.

It will be readily appreciated that the roles of moving source XS and stationary detector DM may be reversed, and application scenarios are contemplated herein, where it is the detector DM that is moved relative to a fixed source. The above fiber-based principles apply equally to such embodiments. In Figs. 8,9, no fiber F1 is needed at patient PAT, who may remain, as said, stationery in this embodiment, such as may be useful for imaging elderly or bedridden patients. Again, if source XS or detector DM remains stationary (at least there is no inclination), and only one component XS, DM is moved, no fiber is needed at the stationary component DM, XS. Thus, in some embodiments, it is source XS that is stationary, and it is detector DM that is moved around patient in an arc or lineal or in other scan path, although lineal or in arc is preferred as the tomographic processing is greatly simplified. In either case, the manner and course of movement should be preferably predefined.

This movement may be done by clinical staff who moves past patient in an arc around patient, whilst holding the detector with its radiation sensitive surface towards patient PAT. User may need to don special X-radiation-shielding protective hazard gear, which may be impractical in most settings. Thus, moving the source XS instead by staff and walking this in an arc or straight line around or past patient is preferred herein, as described above.

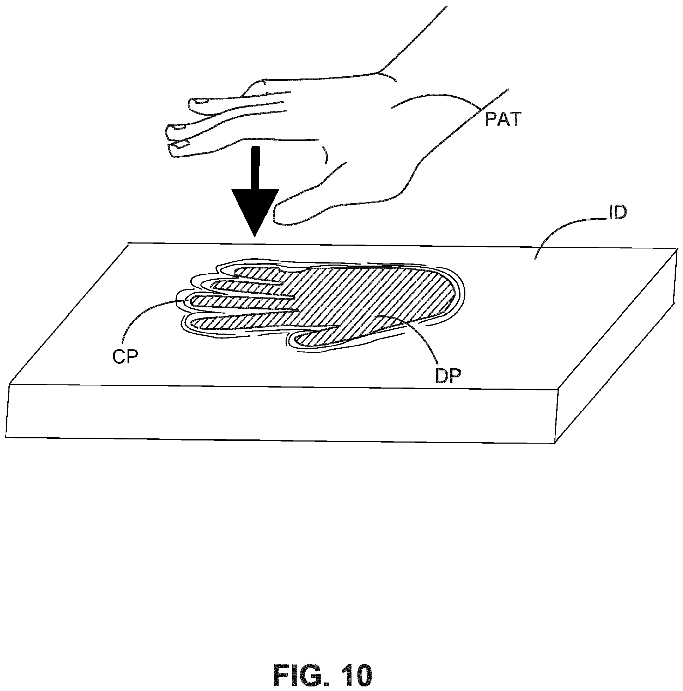

Fig. 10 shows an immobilization device ID that may be used in embodiments of Figs. 9 or similar, where patient PAT is supposed to lie still whilst the source XS is passed the patient. The immobilization device ID may be arranged as a mattress or as a pillow, cushion type object, fashioned from thermoplastics foam. Such foams respond with deformation if contacted, lied thereon. On contact, foam material displaced and forms a depression DP, a negative mold case of the contacting anatomy, such as patient's hand as illustrated. The depression DP conforms to the shape of the body part, and a surrounding slightly raised, crater CR like material border is formed. The foam hardens in this displaced state, with the remaining depression DP capable of securely holding the body part into place during imaging. The immobilizer device ID may be arranged as mattress on patient support PS, for example or table arranged in the examination region between the X-ray source and the detector. Viscoelastic polyurethane foam (memory foam) may be used, adapted to patients surface due to heat and pressure.

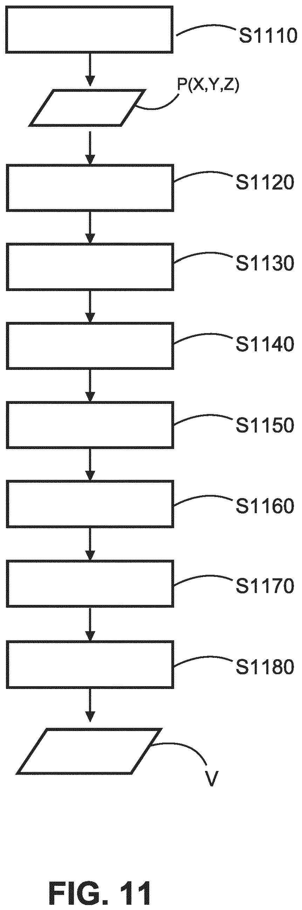

Reference is now made to Fig. 11, which shows a flow chart of a computer implemented enabling method for tomographic imaging with radiography X-ray imaging setups, based on 3D measurements obtained from optical fiber including sensors, and arranged at the patient. Optionally, the optical fiber or a second optical fiber is arranged at the detector. As a further option, the optical fiber is arranged at the detector and/or at the X-ray source of the radiography imaging apparatus.

The radiography imaging apparatus is not of the rotational type, in particular, it does not include a rotatable gantry with source and detector rotatable around ROI/patient, and/or does not include rotational encoders on a journalled shaft. In short, the proposed method allows tomographic imaging with simple imaging equipment. In extreme cases, a fixed X-ray detector opposite a fixed X-ray source with patient in between is sufficient for the proposed method. In this case, the optical fiber at the patient measures 3D positions that represent patient's pose, e.g., patient turning about their longitudinal axis whilst being imaged to acquire projection data. The 3D positional data from the optical fiber system can be converted into angular projection data that represents changing poses of patient relative to source or detector, which can be used together with the acquired projection data for tomographic reconstruction of slice or volume of the ROI.

The patient motion may be rotational or may instead be lineal, the latter in particular for tomosynthetic reconstruction. However, patient motion is not necessarily required in all embodiments herein. Instead of patient moving, it is the source or detector that is moved by motor or manually by user relative to patient/ROI. Preferably, it is the source that is moved lineal past ROI or on an arc around ROI, with less than 180°, 180°, or more than 180° angular coverage, as required. In case of no patient motion, optical fiber is arranged at source or detector, or at both connecting source and detector, to measure, in particular pose of source relative to patient, which can again be converted into projection direction data for use in tomographic reconstruction. Imaging may be done during such relative patient vs source or detector motion or may be done in a "step & shoot" fashion, before and after incremental pose change.

Arrangement of fiber on/at patient, or on/at detector or source may be done in a plane-defining manner with the sensors in fiber defining at least 3 reference points and thus a respective plane at patient, detector or source. Angles between normals of such planes, such as patient plane vs detector plane, or of detector plane vs source plane, represent angular projection direction data for use in tomographic reconstruction.

At step S1110, such 3D positional measurements are obtained based on light sent down optical fiber arranged at/on patient's body. Optical fiber may be arranged in a plane-defining manner, for example, on patient's back, or on other anatomy. Optionally, the same or different fiber is arranged on detector to define a corresponding plane there for detector's radiation sensitive surface. In addition or instead, optical fiber may be arranged in similar manner at the X-ray source.

At step S1120, based on such 3D position measurements, pose information is computed that represents patient's pose relative to detector or source.

In some embodiments of step S1120, 3D positional measurements define the planes and a respective normal thereto are computed, one for the patient plane, and one for the detector plane. However, the detector plane may be pre-defined in case of stationary detector module so in that case no sensor measurements may be received from there as the detector normal is a design constant.

In step S1130, projection direction data is computed based on the 3D position measurements. In particular, deviation angle between two normals is computed, based on analytical geometric techniques to so define a projection direction for given instant.

At step S1140 either before step S1120 or thereafter a projection image ("frame" at instant t) is obtained. In the preferred embodiment, but optional herein, the acquisition or projection data in case of a (mobile) X-ray unit IA is breath-gated to avoid parallax errors in later tomosynthesis. Preferably a "step & shoot" acquisition protocol is used, where steps S1130, S 1140 of projection direction data computation and acquisition are alternated: the patient is asked to move in increments, each increment followed by instances of step S1130, S1140, and so forth. A continuous scheme is not excluded herein, as some tomographic algorithms geared for cone beam /helical geometries are designed to cope with continuous acquisition protocols, and may be suitably adapted for present purposes, as required.

At step S1150 the projection direction data so determined at S1130 is associated with the respective frame acquired at the time. Time stamping in the fiber system and the imager may be used to facilitate association between projection direction data and frame.

The patient is expected to move either self-induced, or by a facilitator such as a swivel stool, during steps S1130, S1140 or before and after any such one or more steps S1130, S1140, to acquire a stream of projection data frames associated with respective projection direction data caused by relative motion/pose changes between patient and detector and/or source.

At optional step S1160, a correction operation may be performed in case movement of patient, if any, is not regular as may be expected in some instances. Such correction may be based on 3D position information of additional optical fiber that is arranged at source.

At step S1170, the data stream of projection imagery and their associated projection direction data is provided for further processing, such as storing, displaying etc.

Preferably, the further processing includes performing a tomographic reconstruction, based on the data stream of projection imagery and their associated projection direction data.

At step S1180 the reconstructed imagery of the ROI, a 3D image volume or a slice in image domain, may then be provided for processing such as storing or visualization on a display device, as required.

As a variant to the above, is may be that pose change is of source relative to stationary patient. The above is equally applicable and practicable, only that the fiber(s) are arranged at source and/or detector, preferably including a fiber that connects the two, and the 3D positional information are used to compute normals of planes at detector and (X-ray) source, and the projection direction data as an angular deviation between the two normal. Again, if detector is stationary, detector normal is a design constant, and need not be determined and no fiber is needed there. Source may be moved manually by user on a frame mounted on a wheeled or tracked undercarriage, etc. Motorization is not excluded, but is not need in most embodiments.

As a further variant to the above, it may in some instances be necessary to let the X-ray source remain stationary, whilst it is the detector module on a similar moveable frame, tracked or wheeled, that is moved past the patient instead of the X-ray source.

Thus, what is mainly exploited herein is a mutual motion between X-ray source and detector connected by one or more optical fibers to capture the positional 3D information which is then converted into the protectional direction data α, as described.

The movement or pose changes as harnessed above may not necessarily be caused by rotation movement by one of patient, source or detector. What matters is that the projection imagery is acquired from multiple directions from multiple viewpoints of the ROI. For example, lineal movements may be sufficient, although the 3D content may then be poorer than imagery acquired in rotational pose changes/movement, and limited angle tomosynthesis may be done instead to full tomographic reconstruction.

Further to the correction step S 1160 as described above, it is expected that patient's rotation intervals are irregular, and the patient may not be placed in the isocenter (as may be assumed by some tomography/tomosynthesis algorithms). However, as the co-ordinates of the patient plane 3D with normal V_{P}, is known, a precise rotation angle can be determined. As mentioned, deviation angle between patient vector VP and the detector normal and/or the beam XB/source XS is the projection angle. The latter may be known by using fiber at the source, as needed. 3D position co-ordinates of patient plane and of the plane at source and/or at detector could be used to determine if there is any translation of the patient relative to source X-ray beam XB, in addition to any rotation about a vertical axis of patient. The projection image acquired at step S 1140 can be corrected with a corrective translation, so that the projection image set is equivalent to a set captured with an iso-centric system, albeit with arbitrary projection angles. This step is optional, to improve reconstruction accuracy.

Instead of compensation for incorrect patient movement at step S 1 160, the X-ray source may be tilted so as to remain in alignment with the detector and patient when the X-ray source is moved instead of the patient changing their pose. This correction operation may be based on the deviation/offset V_{P} with respect to V_{R}, and a prospective correction will be performed by rotating the X-ray source. In this example, as the patient remains stationary, patient normal V_{P} is a fixed constant that can be readily determined.

The components of enabler SYS may be implemented as one or more software modules, run on one or more general-purpose processing units PU such as a workstation associated with the imager IA or on a server computer associated with a group of imagers.

Alternatively, some or all components of system SYS may be arranged in hardware such as a suitably programmed microcontroller or microprocessor, such an FPGA (field-programmable-gate-array) or as a hardwired IC chip, an application specific integrated circuitry (ASIC), integrated into the imaging system IA. In a further embodiment still, system SYS may be implemented in both, partly in software and partly in hardware.

The different components of system SYS may be implemented on a single data processing unit PU. Alternatively, some or more components are implemented on different processing units PU, possibly remotely arranged in a distributed architecture and connectable in a suitable communication network such as in a cloud setting or client-server setup, etc.

One or more features described herein can be configured or implemented as or with circuitry encoded within a computer-readable medium, and/or combinations thereof. Circuitry may include discrete and/or integrated circuitry, a system-on-a-chip (SOC), and combinations thereof, a machine, a computer system, a processor and memory, a computer program.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium (in particular, but not necessarily, a non-transitory medium), such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope. Such reference signs may be comprised of numbers, of letters or any of alphanumeric combination.

## Claims

1. A system (SYS) for facilitating tomographic imaging with a non-rotational radiography imaging apparatus (IA), comprising:
input interface (IN) for receiving, when the system is in use and whilst there is relative motion between patient (PAT) to be imaged and a detector (DM) or X-ray source (XS) of the imaging apparatus, i) 3D position measurement readings acquired by an optical fiber-based system (OFS) via a set of optical-fiber based sensors (Si) arranged at i.1) the patient (PAT) or i.2) at detector (DM) and/or source (XS), wherein the measurement readings are relatable to poses assumable by the patient during motion relative to the detector or to poses of the source (XS) or detector (DM) relative to patient, and ii) projection imagery (λ) acquirable by the imaging apparatus of the patient during, or before and after, such relative motion;
a projection direction determiner (PDD) configured to determine projection direction data (α) for the projection imagery (λ) based on the 3D position measurement readings (P), and
an output interface (OUT) for providing, for reconstruction, the projection imagery associated with the projection direction data.

2. The system of claim 1, comprising a reconstructor (RECON) configured to implement a tomographic reconstruction algorithm to reconstruct a tomographic image of a region of interest of the patient, based on the projection imagery and the projection direction data associated therewith.

3. The system of any one of the preceding claims, wherein the set of optical-fiber based sensors (Si) is integrated at least in part in a wearable (WB) worn by the patient during imaging.

4. The system of any one of the preceding claims, wherein the said relative patient motion is self-induced by patient, or is induced by a rotational facilitator (RF) on or at which the patient resides during imaging, or is induced by at least the X-ray source (XS) or the detector (DM) of the imaging apparatus being moved past the patient.

5. The system of claim 4, wherein the said X-ray source is tiltable so as to maintain alignment with the detector whilst the X-ray source is moved past the patient.

6. The system of claim 4 or 5, wherein the rotational facilitator (RF) includes any one of: a swivel stool, a patient bed.

7. The system of any one of the preceding claims, wherein the projection direction data are determined based on a patient pose normal vector (V_{P}), relative to a normal vector (V_{R}) of a surface of the detector or a normal vector at the source (XS).

8. The system of any one of the preceding claims, wherein the optical-fiber based system (OFS) includes a second set (Sj) of sensors arranged at the detector.

9. The system of any one of the preceding claims, wherein the optical-fiber based system (OFS) includes a second or third set (Sk) of sensors arranged at an X-ray source of the imaging apparatus.

10. The system of any one of the preceding claims, wherein the imaging apparatus (IA) is of the mobile type with its X-ray source (XS) arranged on a wheeled or tracked frame (FR), wherein the imaging apparatus including its X-ray source is movable past the patient on its frame during imaging.

11. An imaging arrangement (IAR) including the imaging apparatus (IA) and further including i) at least the set of optical-fiber based sensor at the detector and/or at the X-ray source, for use with a system of any one of the preceding claims, and/or ii) further including at least the set (S1) of optical-fiber (F1) sensors at the patient (PAT), and/or iii) including the optical fiber-based system (OFS).

12. The imaging arrangement of claim 11, further including the system of any of the preceding claims.

13. A computer-implemented method for facilitating tomographic imaging with a non-rotational radiography imaging apparatus; comprising:
receiving (S1110), when the system is in use and whilst there is relative motion between patient (PAT) to be imaged and a detector (DM) or X-ray source (XS) of the imaging apparatus, i) 3D position measurement readings acquired by an optical fiber-based system (OFS) via a set of optical-fiber based sensors (Si) arranged at i.1) the patient (PAT) or i.2) at detector (DM) and/or source (XS), wherein the measurement readings are relatable to poses assumable by the patient during motion relative to the detector or to poses of the source (XS) or detector (DM) relative to patient, and ii) projection imagery (λ) acquirable by the imaging apparatus of the patient during, or before and after, such relative motion;
determining (S1130) projection direction data (α) for the projection imagery (λ) based on the 3D position measurement readings (P), and
providing (S1170), for reconstruction, the projection imagery associated with the projection direction data.

14. A computer program element, which, when being executed by at least one processing unit, is adapted to cause the processing unit to perform the method as per claim 13.

15. At least one computer readable medium having stored thereon the program element of claim 14.
